# EUROPEAN PATENT SPECIFICATION

(11) **EP 0 405 867 B1**
(45) Date of publication and mention of the grant of the patent: **15.03.1995**
(21) Application number: 90306882.3
(22) Date of filing: 22.06.1990
(51) Int. Cl.: C12N 15/38, A61K 39/25, C12N 15/86

(54) **Novel compounds**
Verbindungen
Composés

(30) Priority: 27.06.1989 US 371772
(43) Date of publication of application: 02.01.1991
(73) Proprietor: SMITHKLINE BEECHAM BIOLOGICALS S.A., 1330 Rixensart (BE)
(72) Inventor: Bollen, Alex, B-1711 Itterbeek (BE); Gregoire, Diane, B-1150 Bruxelles (BE); Heinderyckx, Michel, B-6332 Boignée (BE); Jacobs, Paul, B-7806 Lanquesaint (BE); Massaer, Marc, B-1500 Halle (BE)
(74) Representative: Dalton, Marcus Jonathan William

(56) References cited:
- EP-A- 0 192 902
- EP-A- 0 210 931
- EP-A- 0 211 756
- EP-A- 0 244 155
- WO-A-85/04587
- WO-A-89/00196
- JOURNAL OF GENERAL VIROLOGY vol. 67, 1986, pages 1759-1816, Colchester, GB; A.J. DAVISON et al.: "The complete DNA sequence of Varicella-Zoster Virus"
- JOURNAL OF VIROLOGY vol. 57, no. 3, March 1986, pages 1195-1197, Washington, US; A.J. DAVISON et al.: "New common nomenclature for glycoprotein genes of Varicella-Zoster virus and their glycosylated products"

## Description

This invention relates to expression of Varicella-Zoster Virus glycoproteins and to a vaccine comprising an immunoprotective amount of such protein.

Varicella-Zoster Virus (VZV) is a human herpes virus which is the etiological agent of chicken pox (varicella) and shingles (zoster). Varicella results from an initial, or primary infection, usually contracted during childhood which is relatively benign. However, for adults who were not exposed to varicella during childhood, and occasionally to individuals who are immunocomprised, VZV can be life-threatening. Similarly, a VZV infection can be life-threatening to neonates, for the virus is capable of crossing the placenta. With direct contact, varicella is known to be a highly transmissible infectious disease.

Like most Herpes-Viruses, VZV has a tendency to infect some cells in which its development is arrested. After a variable latent period, the Varicella-Zoster (VZ) virus can be released to initiate infection in other cells. This reactivation of the VZ virus causes an estimated 5 million cases of zoster annually (Plotkin et al., Postgrad Med J 61: 155-63 (1985)). Zoster is characterized by inflammation of the cerebral ganglia and peripheral nerves, and it is associated with acute pain. At present, the factors that reactivate the virus are ill defined.

It has been shown that humans vaccinated with attenuated strains of VZV have received protective immunity from VZV infections (Arbeter et al., J. Pediatr 100 886-93 (1982) and Brunell et al., Lancet ii: 1069-72 (1982)). While effective, this method has limitations due to the difficulty of propagating the Varicella-Zoster virus. Considerable effort has been expended to identify antigenic components of the VZ virus. In order to permit development of improved VZ vaccines, especially subunit vaccines, it is important to isolate VZV envelope proteins. Forghani et al. (J Virol, 52:55-62 (1984)), Okuno et al. (Virol, 129:357-68 (1983)) and Keller et al. (J Virol, 52:293-7 (1984)) have identified numerous virus-specific glycoproteins from VZV-infected cells and VZ virions.

In separate but related research, the VZV genome was elucidated by restriction endonuclease analysis. Physical maps of VZV DNA for eleven restriction endonucleases and cloned DNA fragments have been determined (See Ecker et al., Proc Natl Acad Sci USA 79: 156-160 (1982), Straus et al., Proc Natl Acad Sci USA 79:993-7 (1982), Straus et al., J Gen Virol 64:1031-41 (1983) and Davison et al., J Gen Virol 64:1811-1814 (1983)).

As a result of these studies, the scientific knowledge of VZV proliferated. In addition, there was a concomitant proliferation of nomenclature. Davison et al. (J Virol 57:1195-7 (1986)) hosted a workshop to standardize the nomenclature of VZV glycoprotein genes and their gene products. The three most abundant envelope gene products were designated gpl, gpll and gplll, listed in decreasing order of abundance. All three of these glycoproteins, gpl-gplll, have been shown to elicit neutralizing antibodies in vitro.

The entire nucleotide sequence of VZV was disclosed shortly thereafter, by Davison et al. (J Gen Virol, 67:1759-1816, (1986)). This led to the identification of various glycoprotein genes and helped to clarify the precursor-product relationships of various viral glycoproteins. Like other herpesviruses, the VZV genome is quite complex. It contains 70 open reading frames (ORFs), which encode for five possible glycoprotein genes, designated gpl, gpll, gplll, gplV and gpV. Based upon their deduced amino acid sequence, they have varying degrees of homology to herpes simplex virus (HSV-1) glycoproteins gE, gB, gH, gl and gC, respectively (Davison et al., ld. and Longnecker et al., Proc Natl Acad Sci USA 84:4303-4307 (1987). It is not known if the amino acid homology between the VZV and HSV-1 glycoproteins signifies a functional relationship. While considerable data is available on HSV-1 glycoprotein function, only limited studies of VZV glycoprotein function exist. These studies have focused primarily on glycoproteins involved in virus neutralization (Davison et al., supra, Keller et al., supra and Vafai et al., J Virol 52:953-9 (1984)).

Ellis et al. (U.S. Patent 4,769,239) disclose isolation of two gpl coding sequences from VZV mRNA. Ellis et al. disclose the sequence of a polypeptide containing gpl plus an additional 38 codons found on the amino terminal end. Ellis et al. also disclose a molecule which encodes a 300 amino acid fragment of gpl, whereas native gpl has 623 amino acids.

Ellis et al., J Virol, 53:81-88 (1985), disclose two approaches to map the gpl gene. Small fragments of randomly digested VZV DNA were inserted into a bacterial expression vector. Bacterial colonies transformed by this vector library were screened for antigen expression of gpl as a gpl/lacZ fusion. This hybrid protein was recognised by monoclonal antibodies (mAb) raised to gpl. In addition, mRNA from VZV-infected cells was hybrid-selected by a set of VZV recombinant plasmids and translated in vitro. The resultant products were immunoprecipitated by convalescent zoster serum to gpl.

Ellis et al. (U.S. Patent 4,686,101 and U.S. Patent 4,812,559) disclose the gpll VZV sequence. Ellis et al. also disclose purification of gpll from MRC-5 human diploid fibroblasts infected with the VZ virus. In addition, purified VZV gpll elicited antibodies in guinea pigs that were neutralising when tested in vitro.

Keller et al., Virology, 152:181-191 (1986) suggest that mature gpll is a disulfide-linked heterodimer which is generated by an in vivo proteolytic cleavage in the host cell.

Keller et al. (EP-A-244,155) disclose a DNA fragment coding for gplll, and the purification of gplll from human diploid fibroblasts infected with the VZ virus. Antisera to gplll was shown to neutralize VZV in vitro.

None of the above mentioned references, however, teach or suggest how to produce VZV glycoproteins in clinically useful amounts. Efforts have been made to develop alternative expression systems. Cabriac et al., Virus Res, 10:205-14 (1988) disclose a recombinant vaccinia virus expressing gpl which was localised on the membrane of recombinant virus-infected cells. However, Cabriac et al. do not disclose a method of producing gpl which can be easily purified.

DiNocera et al., Proc Natl Acad Sci USA, 80:7095-8 (1983), disclose transient expression of foreign genes in cultured Drosophila cells. The recombinant plasmids disclosed are under the control of the Drosophila heat shock protein 70 (hsp 70) or Copia promoters.

Bourouis et al., Embo J, 2:1099-1104 (1983), disclose integration of foreign genes into the genome of cultured Drosophila cells. In the baculovirus expression system, a strong, temporally-regulated promoter can be used to express some heterologous genes. Smith et al. (US Patent 4,745,051) and Matsuura et al. (J Gen Virol, 68:1233-50 (1987)) disclose baculovirus vectors containing the polyhedrin gene promoter to express certain prokaryotic and eukaryotic genes.

Frazer et al. (PTC/W088/07082) teach a polyhedrin fusion protein which forms occlusion bodies. It was shown that a polyhedrin-influenza fusion protein expressed an epitope of the hemagglutinin protein which was recognized by antibodies to the influenza virion.

Miller et al. (PCT/W088/02030) teach a method for producing heterologous genes by employing a mixture of at least two genetically distinct baculoviruses.

In addition, certain viral antigens derived from recombinant baculovirus infected insect cells have been demonstrated to be antigenically and immunogenically similar to their native counterparts. Cochrane et al. (EP-A-265,785) and Rusche et al. (EP-A-272,858) disclose the cloning and expression of human immunodeficiency virus (HIV) envelope glycoproteins, gp120 and gp160.

Cochran et al. (EP-A-228,036) disclose expression of vaccinia growth factor (VGF) in a recombinant baculovirus. In addition, Cochran et al. present a hypothetical list of proteins which may be expressed in a recombinant baculovirus, including the hepatitis B surface antigen and Plasmodium polypeptides.

Bishop et al. (EP-A-260,090) and Takehara et al. (J Gen Virol, 69:2763-77 (1988)) disclose expression of Hepatitis B surface antigens in baculoviruses.

Estes et al., (EP-A-273,366) disclose expression of rotavirus genes in a baculovirus system.

Jacobs et al. (U.S. Patent Application Serial No.: 07/287934 and PCT/US89/05550) disclose expression of Plasmodium Circumsporozoite Proteins in a baculovirus system.

In one aspect this invention provides an improved varicella-zoster vaccine consisting of antigenic glycoproteins expressed in insect cells.

This invention further provides a protein from the Varicella-Zoster Virus glycoproteins consisting of anchor-less gpl, anchor-less gpll or anchor-less gplll.

In related aspects, the invention provides: a DNA sequence which codes for said anchor-less glycoprotein; a replicable expression vector capable, in a host cell, of expressing the DNA sequence; a host cell transformed with said replicable expression vector; a vaccine comprising an immunoprotective amount of said anchor-less glycoprotein; a method for protecting a human against Varicella-Zoster Virus infection, which comprises administering a safe and effective amount of said vaccine; use of said anchor-less glycoprotein for the manufacture of the vaccine and said anchor-less glycoprotein for use in the vaccine.

The complete varicella-zoster virus (VZV) nucleotide sequence is disclosed by Davison et al., J Gen Virol, 67:1759-1816 (1986). The DNA sequences of VZV glycoproteins (gp) I, II and III, and their deduced amino acid sequences, are described below:

gp I DNA and amino acid sequence

M G T V N K P V V G V L M G F G I I T G T L R I T 25
CGAATTGCGTGGTTTTAAGTGACTATATTCCGAGGGTCGCCTGTAATATGGGGACAGTTAATAAACCTGTGGTGGGGGTATTGATGGGGTTCGGAATTATCACGGGAACGTTGCGTATAA 115880

N P V R A S V L R Y D D F H T D E D K L D T N S V Y E P Y Y H S D H A E S S W V 65
CGAATCCGGTCAGAGCATCCGTCTTGCGATACGATGATTTTCACACCGATGAAGACAAACTGGATACAAACTCCGTATATGAGCCTTACTACCATTCAGATCATGCGGAGTCTTCATGGG 116000

N R G E S S R K A Y D H N S P Y I W P R N D Y D G F L E N A H E H H G V Y N Q G 105
TAAATCGGGGAGAGTCTTCGCGAAAAGCGTACGATCATAACTCACCTTATATATGGCCACGTAATGATTATGATGGATTTTTAGAGAACGCACACGAACACCATGGGGTGTATAATCAGG 116120

R G I D S G E R L M Q P T Q M S A Q E D L G D D T G I H V I P T L N G D D R H K 145
GCCGTGGTATCGATAGCGGGGAACGGTTAATGCAACCCACACAAATGTCTGCACAGGAGGATCTTGGGGACGATACGGGCATCCACGTTATCCCTACGTTAAACGGCGATGACAGACATA 116240

I V N V D Q R Q Y G D V F K G D L N P K P Q G Q R L I E V S V E E N H P F T L R 185
AAATTGTAAATGTGGACCAACGTCAATACGGTGACGTGTTTAAAGGAGATCTTAATCCAAAACCCCAAGGCCAAAGACTCATTGAGGTGTCAGTGGAAGAAAATCACCCGTTTACTTTAC 116360

A P I Q R I Y G V R Y T E T W S F L P S L T C T G D A A P A I Q H I C L K H T T 225
GCGCACCGATTCAGCGGATTTATGGAGTCCGGTACACCGAGACTTGGAGCTTTTTGCCGTCATTAACCTGTACGGGAGACGCAGCGCCCGCCATCCAGCATATATGTTTAAAACATACAA 116480

C F Q D V V V D V D C A E N T K E D Q L A E I S Y R F Q G K K E A D Q P W I V V 265
CATGCTTTCAAGACGTGGTGGTGGATGTGGATTGCGCGGAAAATACTAAAGAGGATCAGTTGGCCGAAATCAGTTACCGTTTTCAAGGTAAGAAGGAAGCGGACCAACCGTGGATTGTTG 116600

N T S T L F D E L E L D P P E I E P G V L K V L R T E K Q Y L G V Y I W N M R G 305
TAAACACGAGCACACTGTTTGATGAACTCGAATTAGACCCCCCCGAGATTGAACCGGGTGTCTTGAAAGTACTTCGGACAGAAAAACAATACTTGGGTGTGTACATTTGGAACATGCGCG 116720

S D G T S T Y A T F L V T W K G D E K T R N P T P A V T P Q P R G A E F H M W N 345
GCTCCGATGGTACGTCTACCTACGCCACGTTTTTGGTCACCTGGAAAGGGGATGAAAAAACAAGAAACCCTACGCCCGCAGTAACTCCTCAACCAAGAGGGGCTGAGTTTCATATGTGGA 116840

Y H S H V F S V G D T F S L A M H L Q Y K I H E A P F D L L L E W L Y V P I D P 385
ATTACCACTCGCATGTATTTTCAGTTGGTGATACGTTTAGCTTGGCAATGCATCTTCAGTATAAGATACATGAAGCGCCATTTGATTTGCTGTTAGAGTGGTTGTATGTCCCCATCGATC 116960

T C Q P M R L Y S T C L Y H P N A P Q C L S H M N S G C T F T S P H L A Q R V A 425
CTACATGTCAACCAATGCGGTTATATTCTACGTGTTTGTATCATCCCAACGCACCCCAATGCCTCTCTCATATGAATTCCGGTTGTACATTTACCTCGCCACATTTAGCCCAGCGTGTTG 117080

S T V Y Q N C E H A D N Y T A Y C L G I S H M E P S F G L I L H D G G T T L K F 465
CAAGCACAGTGTATCAAAATTGTGAACATGCAGATAACTACACCGCATATTGTCTGGGAATATCTCATATGGAGCCTAGCTTTGGTCTAATCTTACACGACGGGGGCACCACGTTAAAGT 117200

V D T P E S L S G L Y V F V V Y F N G H V E A V A Y T V V S T V D H F V N A I E 505
TTGTAGATACACCCGAGAGTTTGTCGGGATTATACGTTTTTGTGGTGTATTTTAACGGGCATGTTGAAGCCGTAGCATACACTGTTGTATCCACAGTAGATCATTTTGTAAACGCAATTG 117320

E R G F P P T A G Q P P A T T K P K E I T P V N P G T S P L L R Y A A W T G G L 545
AAGAGCGTGGATTTCCGCCAACGGCCGGTCAGCCACCGGCGACTACTAAACCCAAGGAAATTACCCCCGTAAACCCCGGAACGTCACCACTTCTACGATATGCCGCATGGACCGGAGGGC 117440

A A V V L L C L V I F L I C T A K R M R V K A Y R V D K S P Y N Q S M Y Y A G L 585
TTGCAGCAGTAGTACTTTTATGTCTCGTAATATTTTTAATCTGTACGGCTAAACGAATGAGGGTTAAAGCCTATAGGGTAGACAAGTCCCCGTATAACCAAAGCATGTATTACGCTGGCC 117560

P V D D F E D S E S T D T E E E F G N A I G G S H G G S S Y T V Y I D K T R * 624
TTCCAGTGGACGATTTCGAGGACTCGGAATCTACGGATACGGAAGAAGAGTTTGGTAACGCGATTGGAGGGAGTCACGGGGGTTCGAGTTACACGGTGTATATAGATAAGACCCGGTGAT 117680

CACCGAACCGGGGCAACGCCGAGCGTGTAAATTTAAATAAAAAACAGTACGCCCTTATCCGGTGTATGTTTTAAATTTATTTTTTTTTTTCTATATAAAGGGATGGGGTGTCAGGATCTCT 117800

gpIII DNA and amino acid sequences

M F A L V L A V V I L P L W T T 16
GTGGGTGAGGAGGGGTAAAGGCACGCTGTGTGCATAACGTTGCGGTGATATTGTAGCGCAAGTAACAGCGACTATGTTTGCGCTAGTTTTAGCGGTGGTAATTCTTCCTCTTTGGACCAC 66123

A N K S Y V T P T P A T R S I G H M S A L L R E Y S D R N M S L K L E A F Y P T 56
GGCTAATAAATCTTACGTAACACCAACCCCTGCGACTCGCTCTATCGGACATATGTCTGCTCTTCTACGAGAATATTCCGACCGTAATATGTCTCTGAAATTAGAAGCCTTTTATCCTAC 66248

G F D E E L I K S L H W G N D R K H V F L V I V K V N P T T H E G D V G L V I F 96
TGGTTTCGATGAAGAACTCATTAAATCACTTCACTGGGGAAATGATAGAAAACACGTTTTTCTTGTTATTGTTAAGGTTAACCCTACAACACACGAAGGAGACGTCGGGCTGGTTATATT 66368

P K Y L L S P Y H F K A E H R A P F P A G R F G F L S H P V T P D V S F F D S S 136
TCCAAAATACTTGTTATCGCCATACCATTTCAAAGCAGAACATCGAGCACCGTTTCCTGCTGGACGTTTTGGATTTCTTAGTCACCCTGTGACACCCGACGTGAGCTTCTTTGACAGTTC 66488

F A P Y L T T Q H L V A F T T F P P N P L V W H L E R A E T A A T A E R P F G V 176
GTTTGCGCCGTATTTAACTACGCAACATCTTGTTGCGTTTACTACGTTCCCACCAAACCCCCTTGTATGGCATTTGGAAAGAGCTGAGACCGCAGCAACTGCAGAAAGGCCGTTTGGGGT 66608

S L L P A R P T V P K N T I L E H K A H F A T W D A L A R H T F F S A E A I I T 216
AAGTCTTTTACCCGCTCGCCCAACAGTCCCCAAGAATACTATTCTGGAACATAAAGCGCATTTTGCTACATGGGATGCCCTTGCCCGACATACTTTTTTTTCTGCCGAAGCAATTATCAC 66728

N S T L R I H V P L F G S V W P I R Y W A T G S V L L T S D S G R V E V N I G V 256
CAACTCAACGTTGAGAATACACGTTCCCCTTTTTGGGTCGGTATGGCCAATTCGATACTGGGCCACCGGTTCGGTGCTTCTCACAAGCGACTCGGGTCGTGTGGAAGTAAATATTGGTGT 66848

G F M S S L I S L S S G P P I E L I V V P H T V K L N A V T S D T T W F Q L N P 296
AGGATTTATGAGCTCGCTCATTTCTTTATCCTCTGGACCACCGATAGAATTAATTGTTGTACCACATACAGTAAAACTGAACGCGGTTACAAGCGACACCACATGGTTCCAGCTAAATCC 66968

P G P D P G P S Y R V Y L L G R G L D M N F S K H A T V D I C A Y P E E S L D Y 336
ACCGGGTCCGGATCCGGGGCCATCTTATCGAGTTTATTTACTTGGACGTGGGTTGGATATGAATTTTTCAAAGCATGCTACGGTCGATATATGCGCATATCCCGAAGAGAGTTTGGATTA 67088

R Y H L S M A H T E A L R M T T K A D Q H D I N E E S Y Y H I A A R I A T S I F 376
CCGCTATCATTTATCCATGGCCCACACGGAGGCTCTGCGGATGACAACGAAGGCGGATCAACATGACATAAACGAGGAAAGCTATTACCATATCGCCGCAAGAATAGCCACATCAATTTT 67208

A L S E M G R T T E Y F L L D E I V D V Q Y Q L K F L N Y I L M R I G A G A H P 416
TGCGTTGTCGGAAATGGGCCGTACCACAGAATATTTTCTGTTAGATGAGATCGTAGATGTTCAGTATCAATTAAAATTCCTTAATTACATTTTAATGCGGATAGGAGCAGGAGCTCATCC 67328

N T I S G T S D L I F A D P S Q L H D E L S L L F G Q V K P A N V D Y F I S Y D 456
CAACACTATATCCGGAACCTCGGATCTGATCTTTGCCGATCCATCGCAGCTTCATGACGAACTTTCACTTCTTTTTGGTCAGGTAAAACCCGCAAATGTCGATTATTTTATTTCATATGA 67448

E A R D Q L K T A Y A L S R G Q D H V N A L S L A R R V I M S I Y K G L L V K Q 496
TGAAGCCCGTGATCAACTAAAGACCGCATACGCGCTTTCCCGTGGTCAAGACCATGTGAATGCACTTTCTCTCGCCAGGCGTGTTATAATGAGCATATACAAGGGGCTGCTTGTGAAGCA 67568

N L N A T E R Q A L F F A S M I L L N F R E G L E N S S R V L D G R T T L L L M 536
AAATTTAAATGCTACAGAGAGGCAGGCTTTATTTTTTGCCTCAATGATTTTATTAAATTTCCGCGAAGGACTAGAAAATTCATCTCGGGTATTAGACGGTCGCACAACTTTGCTTTTAAT 67688

T S M C T A A H A T Q A A L N I Q E G L A Y L N P S K H M F T I P N V Y S P C M 576
GACATCCATGTGTACGGCAGCTCACGCCACGCAAGCAGCACTTAACATACAAGAAGGCCTGGCATACTTAAATCCTTCAAAACACATGTTTACAATACCAAACGTATACAGTCCTTGTAT 67808

G S L R T D L T E E I H V M N L L S A I P T R P G L N E V L H T Q L D E S E I F 616
GGGTTCCCTTCGTACAGACCTCACGGAAGAGATTCATGTTATGAATCTCCTGTCGGCAATACCAACACGCCCAGGACTTAACGAGGTATTGCATACCCAACTAGACGAATCTGAAATATT 67928

D A A F K T M M I F T T W T A K D L H I L H T H V P E V F T C Q D A A A R N G E 656
CGACGCGGCATTTAAAACCATGATGATTTTTACCACATGGACTGCCAAAGATTTGCATATACTCCACACCCATGTACCAGAAGTATTTACGTGTCAAGATGCAGCCGCGCGTAACGGAGA 68048

Y V L I L P A V Q G H S Y V I T R N K P Q R G L V Y S L A D V D V Y N P I S V V 696
ATATGTGCTCATTCTTCCAGCTGTCCAGGGACACAGTTATGTGATTACACGAAACAAACCTCAAAGGGGTTTGGTATATTCCCTGGCAGATGTGGATGTATATAACCCCATATCCGTTGT 68168

Y L S R D T C V S E H G V I E T V A L P H P D N L K E C L Y C G S V F L R Y L T 736
TTATTTAAGCAGGGATACTTGCGTGTCTGAACATGGTGTCATAGAGACGGTCGCACTGCCCCATCCGGACAATTTAAAAGAATGTTTGTATTGCGGAAGTGTTTTTCTTAGGTATCTAAC 68288

T G A I M D I I I I D S K D T E R Q L A A M G N S T I P P F N P D M H G D D S K 776
CACGGGGGCGATTATGGATATAATTATTATTGACAGCAAAGATACAGAACGACAACTAGCCGCTATGGGAAACTCCACAATTCCACCCTTCAATCCAGACATGCACGGGGATGACTCTAA 68408

A V L L F P N G T V V T L L G F E R R Q A I R M S G Q Y L G A S L G G A F L A V 816
GGCTGTGTTGTTGTTTCCAAACGGAACTGTGGTAACGCTTCTAGGATTCGAACGACGACAAGCCATACGAATGTCGGGACAATACCTTGGGGCCTCTTTAGGAGGGGCGTTTCTGGCGGT 68528

V G F G I I G W M L C G N S R L R E Y N K I P L T 841
AGTGGGGTTTGGTATTATCGGATGGATGTTATGTGGAAATTCCCGCCTTCGAGAATATAATAAAATACCTCTGACATAAAAAACATGTATAATAAAAAGTCACTATAAACGTATTCTCTA 68648

gpII DNA and amino acid sequences

M F V T A V V S V S P S S F Y E S L Q V E P T Q S E D I T R S 31
TGGTTATGCTTTTTATGCATATTTTCTATGTTTGTTACGGCGGTTGTGTCGGTCTCTCCAAGCTCGTTTTATGAGAGTTTACAAGTAGAGCCCACACAATCAGAAGATATAACCCGGTCT 57100

A H L G D G D E I R E A I H K S Q D A E T K P T F Y V C P P P T G S T I V R L E 71
GCTCATCTGGGCGATGGTGATGAAATCAGAGAAGCTATACACAAGTCCCAGGACGCCGAAACAAAACCCACGTTTTACGTCTGCCCACCGCCAACAGGCTCCACAATCGTACGATTAGAA 57220

P T R T C P D Y H L G K N F T E G I A V V Y K E N I A A Y K F K A T V Y Y K D V 111
CCAACTCGGACATGTCCGGATTATCACCTTGGTAAAAACTTTACAGAGGGTATTGCTGTTGTTTATAAAGAAAACATTGCAGCGTACAAGTTTAAGGCGACGGTATATTACAAAGATGTT 57340

I V S T A W A G S S Y T Q I T N R Y A D R V P I P V S E I T D T I D K F G K C S 151
ATCGTTAGCACGGCGTGGGCCGGAAGTTCTTATACGCAAATTACTAATAGATATGCGGATAGGGTACCAATTCCCGTTTCAGAGATCACGGACACCATTGATAAGTTTGGCAAGTGTTCT 57460

S K A T Y V R N N H K V E A F N E D K N P Q D M P L I A S K Y N S V G S K A W H 191
TCTAAAGCAACGTACGTACGAAATAACCACAAAGTTGAAGCCTTTAATGAGGATAAAAATCCACAGGATATGCCTCTAATCGCATCAAAATATAATTCTGTGGGATCCAAAGCATGGCAT 57580

T T N D T Y M V A G T P G T Y R T G T S V N C I I E E V E A R S I F P Y D S F G 231
ACTACCAATGACACGTACATGGTTGCCGGAACCCCCGGAACATATAGGACGGGCACGTCGGTGAATTGCATCATTGAGGAAGTTGAAGCCAGATCAATATTCCCTTATGATAGTTTTGGA 57700

L S T G D I I Y M S P F F G L R D G A Y R E H S N Y A M D R F H Q F E G Y R Q R 271
CTTTCCACGGGAGATATAATATACATGTCCCCGTTTTTTGGCCTACGGGATGGTGCATACAGAGAACATTCCAATTATGCAATGGATCGTTTTCACCAGTTTGAGGGTTATAGACAAAGG 57820

D L D T R A L L E P A A R N F L V T P H L T V G W N W K P K R T E V C S L V K W 311
GATCTTGACACTAGAGCATTACTGGAACCTGCAGCGCGGAACTTTTTAGTCACGCCTCATTTAACGGTTGGTTGGAACTGGAAGCCAAAACGAACGGAAGTTTGTTCGCTTGTCAAGTGG 57940

R E V E D V V R D E Y A H N F R F T M K T L S T T F I S E T N E F N L N Q I H L 351
CGTGAGGTTGAAGACGTAGTTCGCGATGAGTATGCACACAATTTTCGCTTTACAATGAAAACACTTTCTACCACGTTTATAAGTGAAACAAACGAGTTTAATCTTAACCAAATCCATCTC 58060

S Q C V K E E A R A I I N R I Y T T R Y N S S H V R T G D I Q T Y L A R G G F V 391
AGTCAATGTGTAAAGGAGGAAGCCCGGGCTATTATTAACCGGATCTATACAACCAGATACAACTCATCTCATGTTAGAACCGGGGATATCCAGACCTACCTTGCCAGAGGGGGGTTTGTT 58180

V V F Q P L L S N S L A R L Y L Q E L V R E N T N H S P Q K H P T R N T R S R R 431
GTGGTGTTTCAACCCCTGCTGAGCAATTCCCTCGCCCGTCTCTATCTCCAAGAATTGGTCCGTGAAAACACTAATCATTCACCACAAAAACACCCGACTCGAAATACCAGATCCCGACGA 58300

S V P V E L R A N R T I T T T S S V E F A M L Q F T Y D H I Q E H V N E M L A R 471
AGCGTGCCAGTTGAGTTGCGTGCCAATAGAACAATAACAACCACCTCATCGGTGGAATTTGCTATGCTCCAGTTTACATATGACCACATTCAAGAGCATGTTAATGAAATGTTGGCACGT 58420

I S S S W C Q L Q N R E R A L W S G L F P I N P S A L A S T I L D Q R V K A R I 511
ATCTCCTCGTCGTGGTGCCAGCTACAAAATCGCGAACGCGCCCTTTGGAGCGGACTATTTCCAATTAACCCAAGTGCTTTAGCGAGCACCATTTTGGATCAACGTGTTAAAGCTCGTATT 58540

L G D V I S V S N C P E L G S D T R I I L Q N S M R V S G S T T R C Y S R P L I 551
CTCGGCGACGTTATCTCCGTTTCTAATTGTCCAGAACTGGGATCAGATACACGCATTATACTTCAAAACTCTATGAGGGTATCTGGTAGTACTACGCGTTGTTATAGCCGTCCTTTAATT 58660

S I V S L N G S G T V E G Q L G T D N E L I M S R D L L E P C V A N H K R Y F L 591
TCAATAGTTAGTTTAAATGGGTCCGGGACGGTGGAGGGCCAGCTTGGAACAGATAACGAGTTAATTATGTCCAGAGATCTGTTAGAACCATGCGTGGCTAATCACAAGCGATATTTTCTA 58780

F G H H Y V Y Y E D Y R Y V R E I A V H D V G M I S T Y V D L N L T L L K D R E 631
TTTGGGCATCACTACGTATATTATGAGGATTATCGTTACGTCCGTGAAATCGCAGTCCATGATGTGGGAATGATTAGCACTTACGTAGATTTAAACTTAACACTTCTTAAAGATAGAGAG 58900

F M P L Q V Y T R D E L R D T G L L D Y S E I Q R R N Q M H S L R F Y D I D K V 671
TTTATGCCGCTGCAAGTATATACAAGAGACGAGCTGCGGGATACAGGATTACTAGACTACAGTGAAATTCAACGCCGAAATCAAATGCATTCGCTGCGTTTTTATGACATAGACAAGGTT 59020

V Q Y D S G T A I M Q G M A Q F F Q G L G T A G Q A V G H V V L G A T G A L L S 711
GTGCAATATGATAGCGGAACGGCCATTATGCAGGGCATGGCTCAGTTTTTCCAGGGACTTGGGACCGCGGGCCAGGCCGTTGGACATGTGGTTCTTGGGGCCACGGGAGCGCTGCTTTCC 59140

T V H G F T T F L S N P F G A L A V G L L V L A G L V A A F F A Y R Y V L K L K 751
ACCGTACACGGATTTACCACGTTTTTATCTAACCCATTTGGGGCATTGGCCGTGGGATTATTGGTTTTGGCGGGACTGGTAGCGGCCTTTTTTGCGTACCGGTACGTGCTTAAACTTAAA 59260

T S P M K A L Y P L T T K G L K Q L P E G M D P F A E K P N A T D T P I E E I G 791
ACAAGCCCGATGAAGGCATTATATCCACTCACAACCAAGGGGTTAAAACAGTTACCGGAAGGAATGGATCCCTTTGCCGAGAAACCCAACGCTACTGATACCCCAATAGAAGAAATTGGC 59380

D S Q N T E P S V N S G F D P D K F R E A Q E M I K Y M T L V S A A E R Q E S K 831
GACTCACAAAACACTGAACCGTCGGTAAATAGCGGGTTTGATCCCGATAAATTTCGAGAAGCCCAGGAAATGATTAAATATATGACGTTAGTATCTGCGGCTGAGCGCCAAGAATCTAAA 59500

A R K K N K T S A L L T S R L T G L A L R N R R G Y S R V R T E N V T G V 868
GCCCGCAAAAAAAATAAGACTAGCGCCCTTTTAACTTCACGTCTTACCGGCCTTGCTTTACGAAATCGCCGAGGATACTCCCGTGTTCGCACCGAGAATGTAACGGGGGTGTAAATAGCC 59620

The first ATG codes for a N-terminal methionine and the last codon, TGA or TAA, is a translation termination (i.e. stop) signal.

As used herein, the terms 'gpl', 'gpll' or 'gplll' include both the full length membrane glycoproteins of varicella-zoster virus, substantially as illustrated above, and all immunogenic derivatives thereof including anchor-less variants. The term 'immunogenic derivative' encompasses any molecule such as a truncated VZV glycoprotein or other derivatives which retain the ability to induce an immune resonse to VZV following internal administration to man. Such other derivatives can be prepared by the addition, deletion, substitution or rearrangement of amino acids or by chemical modifications thereof.

The VZV glycoproteins of the invention comprise anchor-less glycoprotein derivatives which are missing from 4 to 20 percent of the total amino acid residues at the carboxy terminal end, as discussed more fully below.

As an example, the gpl used in the instant invention is substantially the same (i.e. differs in no more than 10 amino acids) as the protein encoded by the above illustrated DNA sequence, and which is lacking the carboxy terminal anchor region (approximately amino acids 547-623). Similarly, as an example gpll and gplll of the instant invention are substantially the same as disclosed above and are missing amino acids 699-868 (gpll) and 803-841 (gplll) respectively.

A preferred glycoprotein is gpll 1-698.

The immunogenic derivative of the invention can be a hybrid, that is, a fusion polypeptide containing additional sequences which can carry one or more epitopes from other VZV glycoproteins, e.g. gpl-gpll, gpl-gplll, gpll-gplll or gpl-gpll-gplll, other VZV antigens, or other non-VZV antigens. Alternatively, the immunogenic derivative of the invention can be fused to a carrier polypeptide which has immunostimulating properties, as in the case of an adjuvant, or which otherwise enhances the immune response to the VZV glycoprotein, or which is useful in expressing, purifying or formulating the VZV glycoprotein.

In a further aspect, the invention provides a process for preparing an anchor-less VZV glycoprotein according to the invention which process comprises expressing a DNA sequence encoding said glycoprotein in a recombinant host cell and recovering the glycoprotein product.

The process of the invention may be performed by conventional recombinant techniques such as described in Maniatis et. al., Molecular Cloning - A Laboratory Manual; Cold Spring Harbor, 1982 and DNA Cloning vols I, II and III (D.M. Glover ed., IRL Press Ltd).

DNA molecules comprising such coding sequences can be derived from VZV mRNA using known techniques (e.g. making complementary or cDNAs from a mRNA template) or can be isolated from VZV genomic DNA. See Ecker et al., Proc Natl Acad Sci USA 79:156-160 (1982), Straus et al., Proc Natl Acad Sci USA 79:993-7 (1982), Straus et al., J Gen Virol 64:1031-41 (1983) and Davison et al., J Gen Virol 64:1811-1814 (1983). Alternatively the DNA molecules encoding gpl, gpll and gplll can be synthesized by standard DNA synthesis techniques.

The invention thus also provides a process for preparing the DNA sequence by the condensation of appropriate mono-, di- or oligomeric nucleotide units.

The preparation may be carried out chemically, enzymatically, or by a combination of the two methods, in vitro or in vivo as appropriate. Thus, the DNA sequence may be prepared by the enzymatic ligation of appropriate DNA fragments, by conventional methods such as those described by D. M. Roberts et al in Biochemistry 1985, 24, 5090-5098.

The DNA fragments may be obtained by digestion of DNA containing the required sequences of nucleotides with appropriate restriction enzymes, by chemical synthesis, by enzymatic polymerisation, or by a combination of these methods.

Digestion with restriction enzymes may be performed in an appropriate buffer at a temperature of 20°-70 _{°} C, generally in a volume of 50µl or less with 0.1-10µg DNA.

Enzymatic polymerisation of DNA may be carried out in vitro using a DNA polymerase such as DNA polymerase I (Klenow fragment) in an appropriate buffer containing the nucleoside triphosphates dATP, dCTP, dGTP and dTTP as required at a temperature of 10° -37° C, generally in a volume of 50µl or less.

Enzymatic ligation of DNA fragments may be carried out using a DNA ligase such as T4 DNA ligase in an appropriate buffer at a temperature of 4 _{°} C to ambient, generally in a volume of 50µl or less.

The chemical synthesis of the DNA sequence or fragments may be carried out by conventional phosphotriester, phosphite or phosphoramidite chemistry, using solid phase techniques such as those described in 'Chemical and Enzymatic Synthesis of Gene Fragments - A Laboratory Manual' (ed. H.G. Gassen and A. Lang), Verlag Chemie, Weinheim (1982), or in other scientific publications, for example M.J. Gait, H.W.D. Matthes, M. Singh, B.S. Sproat, and R.C. Titmas, Nucleic Acids Research, 1982, 10, 6243; B.S. Sproat and W. Bannwarth, Tetrahedron Letters, 1983, 24, 5771; M.D. Matteucci and M.H Caruthers, Tetrahedron Letters, 1980, 21, 719; M.D. Matteucci and M.H. Caruthers, Journal of the American Chemical Society, 1981, 103, 3185; S.P. Adams et al., Journal of the American Chemical Society, 1983, 105, 661; N.D. Sinha, J. Biernat, J. McMannus, and H. Koester, Nucleic Acids Research, 1984, 12, 4539; and H.W.D. Matthes et al., EMBO Journal, 1984, 3, 801. Preferably an automated DNA synthesizer is employed.

The DNA sequence is preferably prepared by ligating two or more DNA molecules which together comprise a DNA sequence encoding the glycoprotein.

The DNA molecules may be obtained by the digestion with suitable restriction enzymes of vectors carrying the required coding sequences.

The precise structure of the DNA molecules and the way in which they are obtained depends upon the structure of the desired glycoprotein product. The design of a suitable strategy for the construction of the DNA molecule coding for the glycoprotein is a routine matter for the skilled worker in the art.

The expression of the DNA sequence encoding the anchor-less glycoprotein in a recombinant host cell may be carried out by means of a replicable expression vector capable, in the host cell, of expressing the DNA sequence.

The replicable expression vector may be prepared in accordance with the invention, by cleaving a vector compatible with the host cell to provide a linear DNA segment having an intact replicon, and combining said linear segment with one or more DNA molecules which, together with said linear segment, encode the anchor-less glycoprotein, under ligating conditions. The ligation of the linear segment and more than one DNA molecule may be carried out simultaneously or sequentially as desired. Thus, the DNA sequence may be preformed or formed during the construction of the vector, as desired.

The choice of vector will be determined in part by the host, which may be a prokaryotic such as, but not limited to, E. coli or Streptomyces, or a eukaryotic cell, such as, but not limited to, mouse C127, mouse myeloma, HeLa, Chinese Hamster Ovary (CHO), BHK, HaK, COS, yeast (e.g. Pichia pastoris, S. cerevisiae and Hansenula sp.) and insect cells. The host may also be a transgenic animal. Preferably, the host cell is of mammalian or insect origin. More specifically, the preferred host cell of the invention is a CHO, Lepidoptera or Drosophila cell. Suitable vectors for the host cell of the invention include plasmids, bacteriophages, cosmids and recombinant viruses, derived from, for example, baculoviruses and poxviruses such as vaccinia.

The preparation of the replicable expression vector may be carried out conventionally with appropriate enzymes for restriction, polymerisation and ligation of the DNA, by procedures described in, for example, Maniatis et al., cited above. Polymerisation and ligation may be performed as described above for the preparation of the DNA polymer. Digestion with restriction enzymes may be performed in an appropriate buffer at a temperature of 200 -70 0 C, generally in a volume of 50µl or less with 0.1-10µg DNA.

The recombinant host cell is prepared, in accordance with the invention, by transforming a host cell with a replicable expression vector of the invention under transforming conditions. Suitable transforming conditions are conventional and are described in, for example, Maniatis et al., cited above, or "DNA Cloning" Vol. II, D.M. Glover ed., IRL Press Ltd, 1985.

The choice of transforming conditions is determined by the host cell. Thus, a bacterial host such as E. coli may be treated with a solution of CaC1₂ (Cohen et al, Proc. Nat. Acad. Sci., 1973, 69, 2110) or with a solution comprising a mixture of RbCI, MnC1₂, potassium acetate and glycerol, and then with 3-[N-morpholino]-propane-sulphonic acid, RbCI and glycerol. Mammalian cells in culture may be transformed by calcium co-precipitation of the vector DNA onto the cells.

Culturing the transformed host cell under conditions permitting expression of the DNA sequence is carried out conventionally, as described in, for example, Maniatis et al and "DNA Cloning" cited above. Thus, preferably the cell is supplied with nutrient and cultured at a temperature below 45 _{°} C.

The anchor-less glycoprotein expression product is recovered by conventional methods according to the host cell. Thus, where the host cell is bacterial, such as E. coli it may be lysed physically, chemically or enzymatically and the protein product isolated from the resulting lysate. Where the host cell is mammalian, the product may generally be isolated from the nutrient medium.

The DNA sequence may be assembled into vectors designed for isolation of stable transformed mammalian cell lines expressing the anchor-less glycoprotein; e.g. bovine papillomavirus vectors or amplified vectors in Chinese hamster ovary cells (DNA cloning Vol.ll D.M. Glover ed. IRL Press 1985; Kaufman, R.J. et al., Molecular and Cellular Biology 5, 1750-1759, 1985; Pavlakis G.N. and Hamer, D.H., Proceedings of the National Academy of Sciences (USA) 80, 397-401, 1983; Goeddel, D.V. et al., European Patent Application No. 0093619, 1983).

A variety of insect cells and expression systems are also available for expression of heterologous proteins. Insect hosts typically include Drosophila and Lepidoptera cells. In general, these systems employ a recombinant DNA molecule comprising a coding sequence for the gene of interest, under control of a regulatory element, and a selectable marker. A regulatory element is a DNA region or regions which comprises functions necessary or desirable for transcription or translation.

Insect cells which can be used in the invention include Drosophila S1, S2, S3, KC-0 and D.hydei cells. See for example, Schneider et al., J Embryol Exp Morph, 27:353 (1972); Schultz et al., Proc Natl Acad Sci USA, 183:9428 (1986); Sinclair et al., Mol Cell Biol, 5:3208 (1985). Drosophila cells are transfected by standard techniques, including calcium phosphate precipitation, electroporation and viral transfection. Cells are cultured in accordance with standard cell culture procedures in a variety of nutrient media, including e.g., M3 media which consists of balanced salts and essential amino acids. See, Lindquist et al., DIS - (Drosophila Information Services), 58:163 (1982).

Promoters known to be useful in Drosophila include mammalian cell promoters as well as Drosophila promoters, the latter being preferred. Examples of useful Drosophila promoters include the Drosophilametallothionein promoters, the 70 kilodalton heatshock protein promoter (HSP70) and the COPIA LTR. See, for example, DiNocera et al., Proc Natl Acad Sci USA, 80:7095 (1983); McGarry et al., Cell, 42:903 (1985); Johansen et al., European Patent Application No. 88 304093.3 (EP-A-290261).

In one embodiment of this invention, the VZV glycoproteins are expressed in Lepidoptera cells to produce immunogenic proteins. For expression of VZV glycoproteins in Lepidoptera cells, the use of a baculovirus expression system is preferred. In such system, an expression cassette comprising the VZV protein coding sequence, operatively linked (i.e. under control) to a baculovirus promoter, typically is placed into a shuttle vector. Such vector contains a sufficient amount of bacterial DNA to propagate the shuttle vector in E. coli or some other suitable prokaryotic host. Such shuttle vector also contains a sufficient amount of baculovirus DNA flanking the VZV glycoprotein coding sequence so as to permit recombination between a wild-type baculovirus and the heterologous gene. The recombinant vector is then cotransfected into Lepidoptera cells with DNA from a wild-type baculovirus. The recombinant baculoviruses arising from homologous recombination are then selected and plaque purified by standard techniques. See Smith et al., TAES Bull (Texas Agricultural Experimental Station Bulletin) NR 1555, May, 1987.

Promoters for use in Lepidoptera cells include promoters from a baculovirus genome. The promoter of the polyhedrin gene is preferred because the polyhedrin protein is naturally overexpressed relative to other baculovirus proteins. The preferred polyhedrin gene promoter is from the AcMNPV baculovirus. See, Smith et al., US Patent 4,745,051; Smith et al., Proc Natl Acad Sci USA, 82:8404 (1985); and Cochran, EP-A-228,036.

Useful Lepidoptera cells include cells from Trichoplusia ni, Spodoptera frugiperda, Heliothis zea, Autographica californica, Rachiplusia or, Galleria melonella, Manduca sexta or other cells which can be infected with baculoviruses. These include nuclear polyhedrosis viruses (NPV), single nucleocapsid viruses (SNPV) and multiple nucleocapsid viruses (MNPV). The preferred baculoviruses are NPV or MNPV baculoviruses because these contain the polyhedrin gene promoter which is highly expressed in infected cells. Particularly exemplified hereinbelow is the MNPV virus from Autographica california (AcMNPV). However, other MNPV and NPV viruses can also be employed such as the silkworm virus, Bombyx mori. Lepidoptera cells are transfected with the recombinant baculovirus of the invention, according to standard transfection techniques. These include but are not limited to, calcium phosphate precipitation, electroporation, and liposome-mediated transfer. Cells are cultured in accordance with standard cell culture techniques in a variety of nutrient media, including, for example, TC100 (Gibco Europe; Gardiner et al., J Invert Path, 25:363 (1975) supplemented with 10% fetal calf serum (FCS) and grown at 27 _{°} to 28 _{°} C for 18 to 24 hours. Alternatively, serum-free medium that supports insect cell growth has been disclosed by Maiorella et al.(Bio/Technology, 6:1406-1410 (1988) or PCT/W089/01028). Excellent yields of recombinant virus-encoded products are achieved with infection of actively growing cultures at densities of 1 to 1.2 x 10⁶ cells/ml. See, Miller et al., in Setlow and Hollander (eds.), Genetic Engineering: Principles and Methods, Vol 8, p277-98, Plenum Publishing Co., New York, 1986. Murhammer et al., (Bio/Technology, 6:1411-1418 (1988) (or PCT/W089/01029).

The purification of the VZV glycoproteins from cell culture is carried out by conventional protein isolation techniques, e.g. selective precipitation, absorption chromatography, and affinity chromatography including a monoclonal antibody affinity column, as described below.

Production in insect cells can also be accomplished by infecting insect larvae. For example, the VZV glycoproteins can be produced in Heliothis virescens caterpillars by feeding the recombinant baculovirus of the invention along with traces of wild type baculovirus and then extracting the protein from the hemolymph after about two days. See, for example, Miller et al., PCT/W088/02030.

In addition, production of fusion polyhedrin proteins which are capable of forming occlusion bodies are disclosed by Frazer et al., PCT/W088/07082.

This invention also relates to a vaccine containing an immunoprotective amount of VZV glycoprotein(s) according to the invention. The term "immunoprotective" refers to a sufficient amount of VZV glycoprotein-(s), when administered to man, which elicits a protective antibody or immune response against a subsequent VZV infection sufficient to avert or mitigate the disease.

In the vaccine of the invention, an aqueous solution of the VZV glycoprotein(s), can be used directly. Alternatively, the VZV glycoprotein(s), with or without prior lyophilization, can be mixed together or with any of the various known adjuvants. Such adjuvants include, but are not limited to, aluminium hydroxide, muramyl dipeptide and saponins such as Quil A. As a further exemplary alternative, the protein can be encapsulated within microparticles such as liposomes. In yet another exemplary alternative, the VZV glycoprotein(s) can be conjugated to an immunostimulating macromolecule, such as killed Bordetella or a tetanus toxoid.

Vaccine preparation is generally described in New Trends and Developments in Vaccines, Voller et al. (eds.), University Park Press, Baltimore, Maryland, 1978. Encapsulation within liposomes is described by Fullerton, US Patent 4,235,877. Conjugation of proteins to macromolecules is disclosed, for example, by Likhite, US Patent 4,372,954 and Armor et al., US Patent 4,474,757. Use of Quil A is disclosed by Dalsgaard et al., Acta Vet Scand, 18:349 (1977).

The examples which follow are illustrative but not limiting of the invention. Restriction enzymes and other reagents were used substantially in accordance with the vendors' instructions.

### Example 1. Vector Construction

### A)"Anchor-less" gpl: Plasmid pNIV2011

VZV genomic DNA was isolated from a patient suffering from varicella (Professor Rentier, Institut de Pathologie, Universite de Liege, Sart Tilman, Liege, Belgium). The viral DNA was digested with BamHl and a 4 kilobase-pair fragment, corresponding to bases 114,042 to 118,004 (see Davison et al., J Gen Virol, 67: 1759-1816 (1986)), was isolated. This fragment was cloned into the BamHl site of plasmid pUC9, a standard E. coli cloning vector, to create plasmid pNIV2005. Plasmid pNIV2005 enclodes the entire VZV gpl protein of 623 amino acids, including a putative signal sequence, plus 5' and 3' untranslated DNA.

A 1965 base pair Bg11 - Bc1I fragment from pNIV2005 was blunt-ended with DNA polymerase I (i.e. Klenow) and cloned into a blunt-ended Hindlll - Bc1I site of a typical shuttle vector, herein referred to as TigND, to create plasmid pNIV2001.

Plasmid TigND is a derivative of plasmid TND (Connors et al., DNA, 7:651-661 (1988)). Plasmid TigND differs from plasmid TND in that the Rous LTR is replaced by the mouse gamma 2b heavy chain immunoglobin enhancer sequence.

To remove the 5' untranslated DNA, pNIV2001 was digested with Nsplll, Bg1II and Ppuml. Two fragments, a 532 base pair (bp) Nsplll (blunt-ended) - Bg111, and a 7,187 bp Bg1II - Ppuml fragment were isolated. These fragments were ligated with a 741 bp Ppuml - Hindlll (blunt-ended) fragment from plasmid TigND to create plasmid pNIV2002. In pNIV2002, the Hindlll site is recreated 56 base-pairs upstream of the ATG.

From plasmid pNIV2002, a plasmid containing the VZV gpl coding region was constructed which lacks the carboxy terminal anchor sequence but retains a signal sequence. This vector was created by ligating a 861 bp Hindlll - HgiAI fragment (coding for amino acids 1 to 268) to a 817 bp HgiAI - Avail fragment (coding for amino acids 269 to 541), both isolated from pNIV2002, to an Avail - Bc1I synthetic linker, which encodes amino acids 542-546 and a termination codon:

5> GACCGGAGGGCTTGCAT <3
3> GCCTCCCGAACGTACTAG <5

This was ligated into the Hindlll - Bc1I sites of plasmid TndHBB to create vector pNIV2004. (Plasmid TndHBB is a modification of plasmid pTND (cited above) in which Notl sites are introduced flanking the pUC19 sequence.) This vector encodes for amino acids 1 - 546 and is flanked by the restriction sites Hindlll at the 5' end and Bc1 I at the 3' end.

Plasmid pAcYM1 is a baculovirus shuttle vector containing sequences from the AcMNPV genome which includes the polyhedrin gene promoter, but not the polyhedrin gene, and sequences from a high copy bacterial plasmid, pUC8. See Matsuura et al., J Gen Virol, 68:1233-50 (1987).

A 1695 bp Hindlll - Bc1I fragment containing the "anchor-less" gpl sequence was isolated from pNIV2004 and blunt-ended with DNA polymerase I. This fragment was then ligated into a blunt-ended BamHl site of plasmid pAcYMI, which is just 3' to the polyhedrin promoter. This novel plasmid is herein referred to as pNIV2011.

### B) "Anchor-less" gpll: Plasmid pNIV2014

VZV genomic DNA (refer to part A) was digested with the restriction enzyme, EcoRl. A 15 kilobase-pair fragment, corresponding to bases 49,362 to 64,735 (see Davison et al., supra) was isolated, blunt-ended and cloned into the Hindll site of plasmid pUC19, another standard cloning vector. This new plasmid, referred to as pNIV2008, encodes the complete VZV gpll protein of 868 amino acids, which includes a putative signal sequence (amino acids 1-9), plus 5' and 3' untranslated DNA.

To obtain a clone without the untranslated DNA and lacking a carboxy terminal anchor sequence, two more plasmid constructions were needed. The first was to remove 5' untranslated DNA by ligating a Hindlll
- Maelll synthetic linker (which encodes the first two amino acids): 
5> AGCTTACCATGTTT <3
3> ATGGTACAAACAATG <5
 to a 840 bp Maelll - Pstl fragment from pNIV2008 (coding for amino acids 3 to 283), and then ligating this fragment into the Hindlll - Pstl sites of a pUC19 vector. This vector, containing the amino portion of gpll was referred to as clone "A". A 854 Hindlll - Pstl fragment from clone "A" was isolated and ligated to a 1235 bp Pstl - Sacll fragment from pNIV2008 (coding for amino acids 284 to 694) and a synthetic linker, which encodes for amino acids 695-698 and a stop or termination codon:

5> GGGCCAGGCCGTTTAAGTTAACG <3
3> CGCCCGGTCCGGCAAATTCAATTGCTTAA <5

These fragments are then ligated into the Hindlll - EcoRl sites of pUC19 to create clone "C"(pNIV2039). This vector encodes amino acids 1 - 698 of gpll (i.e. including the signal sequence and missing the anchor sequence) and is flanked by the restriction sites Hindlll at the 5' end and Hindll at the 3' end.

A 2108 bp Hindlll - Hindll fragment containing the "anchor-less" gpll sequence was isolated from clone "C" and blunt-ended with DNA polymerase I (i.e. Klenow). This fragment was then ligated into a blunt-ended BamHl site of plasmid pAcYM1, which is just 3' to the polyhedrin promoter. In this plasmid, referred to as pNIV2014, the A of the ATG is located 9 bases downstream from the ligation junction.

### C) "Anchor-less" gplll: Plasmid pNIV2012

A 12.9 Kbp EcoRl fragment (see part B) corresponding to bases 64,735 to 77,656 (see Davison et al., supra) was isolated and ligated into the EcoRl site of pUC9. This resultant plasmid, pNIV2007, encodes the complete VZV gplll protein of 841 amino acids, signal sequence inclusive (the signal sequence is deduced from its hydrophobicity), plus untranslated VZV DNA.

To facilitate further construction, a 4400 bp M1ul fragment was blunt-ended and ligated into a blunt-ended Hindll restriction site of plasmid pUC19. This plasmid is herein referred to as pNIV2003. From pNIV2003, a plasmid containing the coding region of VZV gplll was constructed which lacks the carboxy terminal anchor sequence but contains the signal sequence. Four fragments were ligated into the Hindlll - Bc11 site of plasmid TndHBB (cited above). They comprise a synthetic Hindlll - Avail DNA fragment encoding amino acids 1-13: 
5>AGCTTACCATGTTTGCGCTAGTTTTAGCGGTGGTAATTCTTCCTCTT
TG <3
3>
ATGGTACAAACGCGATCAAAATCGCCACCATTAAGAAGGAGAAACC
TG <5
 a 231 bp Avail - Aatll fragment from pNIV2003 (coding for amino acids 14 to 91), a 2103 bp Aatll - Asull fragment from pNIV2003 (coding for amino acids 92 to 792), and a synthetic Asull - Bc11 fragment which encodes for amino acids 793-802 plus a termination codon: 
5> CGAACGACGACAAGCCATACGAATGTCGGGATGATCTAGAT <3
3> TTGCTGCTGTTCGGTATGCTTACAGCCCTACTAGATCTACTAG <5
 This resultant vector encodes for amino acids 1 - 802 and is flanked by the restriction sites Hindlll at the 5' end and Xbal at the 3' end.

A 2422 bp Hindlll - Xbal fragment containing the "anchor-less" gplll sequence was blunt-ended with DNA polymerase I (i.e. Klenow) and isolated. This fragment was then ligated into a blunt-ended BamHl site of plasmid pAcYM1, which is just 3' to the polyhedrin promoter, to create plasmid pNIV2012.

### Example 2

### Expression in Insect Cells

Spodoptera frugiperda 9 (Sf9) cells are available from the ATCC (Rockville, MD, USA). The Sf9 cells were cotransfected with one of the following recombinant vectors, plasmid pNIV2011, pNIV2014 or pNiV2012 and with wild-type AcMNPV DNA, at 50µg and 1µg respectively; substantially as described by Summers et al., TAES Bull, NR 1555, May 1987, cited above.

Resulting virus particles were obtained by collecting the supernatants. The virus-containing media was then used to infect Sf9 cells in a plaque assay. Subsequent infection of Sf9 cells with a plaque purified recombinant baculovirus resulted in cells expressing the VZV glycoproteins instead of the polyhedrin protein.

The recombinant baculovirus infected cells derived from pNIV2011 were shown to secrete and express a gpl protein, lacking a carboxy terminal anchor sequence. Western blot analysis made with rabbit polyclonal antiserum raised against whole VZV particles showned essentially one band at about 60 kilodaltons.

The recombinant baculovirus infected cells derived from pNIV2014 were shown to express a gpll protein, lacking the carboxy terminal anchor sequence. Western blot analysis made as fo gpl showed essentially two bands of about 63 and 85 kilodaltons.

### Example 3

### Expression of gp 11 in CHO cells

### i) Construction of plasmid pNIV2034

A 2108 bp Hindlll(blunted)-Hindll fragment containing the 'anchor-less' gpll sequence was isolated from pNIV 2039 and introduced between Hindlll (blunted) and EcoRV of plasmid TDN (cited above) to give plasmid pNIV2034.

### 2) Selection of CHO clones expressing gpll s^{±}a-

CHO DHFR-cells were electroporated with pNIV2034. Transformed cells were selected on the basis of their resistance to the antibiotic G418. Spent culture medium as well as cell extracts from about 100 clones for each construction were tested for the presence of gpll. The most promising clones were submitted to amplification with 5nM methotrexate. About 24 clones for each construction were tested again.

### Example 4

### Vaccine Containing VZV Glycoproteins

An illustrative vaccine of this invention is prepared as follows. The recombinant VZV glycoproteins of this invention, such as insect-derived glycoproteins, are added with stirring to a final concentration of 0.1 to 1000 µg/ml polypeptide, preferably 1 to 100 µg/ml, in a buffered saline solution (150mM NaCI, 10mM sodium phosphate pH 6.8; sterilized by filtration) containing 0.5mg A1³⁺ (as aluminium hydroxide gel) per ml. The pH is maintained at pH 6.8 and the mixture is left overnight at about 4 _{°} C. Thimerosal is added to a final concentration of 0.0005%. The pH is checked and adjusted, if necessary, to pH 6.8.

The amount of insect derived VZV glycoprotein present in each vaccine dose is selected as an amount which induces an immunoprotective response without inducing adverse side effects. Such amount will vary depending upon which specific polypeptide is employed and whether or not the vaccine is adjuvanted. An optimal amount for a particular vaccine can be ascertained by standard studies involving observation of antibody titres and other responses in subjects. Following an initial vaccination, subjects will receive booster doses at intervals as necessary to augment and maintain a protective immune response.

The vaccine is preferably administered parenterally, e.g. intramuscularly (im) or subcutaneously (sc), although other routes of administration may be used to elicit a protective response.

The above description and examples fully disclose the invention and the preferred embodiments thereof. The invention is not limited to the embodiments specifically disclosed, but rather encompasses all variations and modifications thereof which come within the scope of the following claims.

## Claims (Claims for the following Contracting State(s) : AT, BE, CH, DE, DK, FR, GB, GR, IT, LI, LU, NL, SE)

1. A protein derived from Varicella Zoster Virus which is one of gpl, gpll or gplll and is missing from 4 to 20 percent of the total amino acid residues of full length glycoprotein at the carboxy terminal end.

2. A protein according Claim 1 selected from gpl 1-546, gpll 1-698 and gplll 1-802 wherein the numbering refers to the amino acid number set forth in the description.

3. A protein according to any of Claims 1 to 2 obtainable from Chinese hamster ovary cells.

4. A DNA sequence which codes for a protein according to any of Claims 1 to 3.

5. A replicable expression vector capable, in a host cell, of expressing the DNA sequence of Claim 4.

6. A host cell transformed with the replicable expression vector of Claim 5.

7. A process for preparing a protein according to any of claims 1 to 3 which process comprises expressing a DNA sequence encoding said protein in a recombinant host cell and recovering the protein product.

8. A protein as claimed in any of claims 1 to 3 for use in medicine.

9. A vaccine composition comprising a protein according to any one of claim 1 to 3 and a pharmaceutically acceptable excipient.

## Claims (Claims for the following Contracting State(s) : ES)

1. A process for preparing a protein derived from Varicella Zoster Virus which is one of gpl, gpll, or gplll, wherein the protein is missing from 4 to 20 percent of the total amino acid residues of full length glycoprotein at the carboxy terminal end, which process comprises expressing a DNA sequence encoding said protein in a recombinant host cell and recovering the protein product.

2. A process according to Claim 1 for preparing a protein selected from gpl 1-546, gpll 1-698 and gplll 1-802 wherein the numbering refers to the amino acid number set forth in the description.

3. A process according to any of Claims 1 or 2 for producing protein in Chinese hamster ovary cells.

4. A process for preparing a vaccine comprising admixing the product of any of claims 1 to 3 with a pharmaceutical excipient.

## Patentansprüche (Patentansprüche für folgende(n) Vertragsstaat(en) : AT, BE, CH, DE, DK, FR, GB, GR, IT, LI, LU, NL, SE)

1. Protein abgeleitet vom Varicella Zoster-Virus, das eines der Proteine gpl, gpll oder gplll ist und dem 4 bis 20 % der gesamten Aminosäurereste des Glykoproteins vollständiger Länge am Carboxyterminus fehlen.

2. Protein nach Anspruch 1, ausqewählt aus gpl 1-546, gpll 1-698 und gplll 1-802, wobei sich die Numerierung auf die Aminosäurenummer bezieht, die in der Beschreibung angegeben ist.

3. Protein nach einem der Ansprüche 1 bis 2, erhältlich aus Ovarialzellen des Chinesischen Hamsters.

4. DNA-Sequenz, die ein Protein nach einem der Ansprüche 1 bis 3 codiert.

5. Replizierbarer Expressionsvektor, der die DNA-Sequenz von Anspruch 4 in einer Wirtszelle exprimieren kann.

6. Wirtszelle, die mit einem replizierbaren Expressionsvektor nach Anspruch 5 transformiert ist.

7. Verfahren zur Herstellung eines Proteins nach einem der Ansprüche 1 bis 3, das die Expression einer DNA-Sequenz, die das Protein codiert, in einer rekombinanten Wirtszelle und die Gewinnung des Proteinprodukts umfaßt.

8. Protein nach einem der Ansprüche 1 bis 3 zur Verwendung in der Medizin.

9. Impfstoff, enthaltend ein Protein nach einem der Ansprüche 1 bis 3 und einen pharmazeutisch verträglichen Excipienten.

## Patentansprüche (Patentansprüche für folgende(n) Vertragsstaat(en) : ES)

1. Verfahren zur Herstellung eines Proteins abgeleitet vom Varicella Zoster-Virus, das eines der Proteine gpl, gpll oder gplll ist, wobei dem Protein 4 bis 20 % der gesamten Aminosäurereste des Glykoproteins vollständiger Länge am Carboxyterminus fehlen, wobei das Verfahren die Expression einer DNA-Sequenz, die das Protein codiert, in einer rekombinanten Wirtszelle und die Gewinnung des Proteinprodukts unfaßt.

2. Verfahren nach Anspruch 1 zur Herstellung eines Proteins, ausgewählt aus gpl 1-546, gpll 1-698 und gplll 1-802, wobei sich die Numerierung auf die Aminosäurenummer bezieht, die in der Beschreibung angegeben ist.

3. Verfahren nach einem der Ansprüche 1 bis 2 zur Herstellung von Proteinen aus Ovarialzellen des Chinesischen Hamsters.

4. Verfahren zur Herstellung eines Impfstoffes, bei dem man das Produkt nach einem der Ansprüche 1 bis 3 mit einem pharmazeutischen Excipienten vermischt.

## Revendications (Revendications pour l'(les) Etat(s) contractant(s) suivant(s) : suivants : AT, BE, CH, DE, DK, FR, GB, GR, IT, LI, LU, NL, SE)

1. Protéine dérivée du Virus Varicella Zoster, qui est une protéine de gpl, gpll ou gplll, et à qui il manque de 4 à 20 p. cent des résidus acides aminés totaux de la glycoprotéine complète, à l'extrémité carboxy terminale.

2. Protéine selon la revendication 1, sélectionnée à partir de gpl 1-546, gpll 1-698, et gplll 1-802, dans lesquels la numérotation se rapporte au nombre d'acides aminés mentionné dans la description.

3. Protéine selon l'une quelconque des revendications 1 à 2, pouvant être obtenue à partir de cellules ovariennes du hamster de Chine.

4. Séquence d'ADN qui code pour une protéine selon l'une quelconque des revendications 1 à 3.

5. Vecteur d'expression réplicable, capable d'exprimer dans une cellule hôte, la séquence d'ADN selon la revendication 4.

6. Cellule hôte transformée avec le vecteur d'expression réplicable selon la revendication 5.

7. Procédé pour la préparation d'une protéine selon l'une quelconque des revendications 1 à 3, lequel procédé comprend l'expression d'une séquence d'ADN encodant ladite protéine dans une cellule hôte recombinante, et la récupération du produit protéique.

8. Protéine selon l'une quelconque des revendications 1 à 3, pour l'utilisation en médecine.

9. Composition pour vaccin comprenant une protéine selon l'une quelconque des revendications 1 à 3, et un excipient pharmaceutiquement acceptable

## Revendications (Revendications pour l'(les) Etat(s) contractant(s) suivant(s) : suivant : ES)

1. Procédé pour la préparation d'une dérivée du Virus Varicella Zoster, qui est une protéine de gpl, gpll ou gplll, et à laquelle protéine manquent de 4 6 20 p. cent des résidus acides aminés totaux de la glycoprotéine complète, à l'extrémité carboxy terminale, lequel procédé comprend l'expression d'une séquence d'ADN encodant ladite protéine dans une cellule hôte recombinante, et la récupération du produit protéique.

2. Procédé selon la revendication 1, pour la préparation d'une protéine sélectionnée à partir de gpl 1-546, gpll 1-698, et gplll 1-802, dans lesquels la numérotation se rapporte au nombre d'acides aminés mentionné dans la description.

3. Procédé selon l'une quelconque des revendications 1 ou 2, pour le production de protéine à partir de cellules ovariennes de hamster de Chine.

4. Procédé pour la préparation d'un vaccin comprenant le mélange d'un produit selon l'une quelconque des revendications 1 à 3, avec un excipient pharmaceutique.
